# FASCICULE DE BREVET EUROPEEN

(11) **EP 0 578 521 B1**
(45) Date de publication et mention de la délivrance du brevet: **21.08.1996**
(21) Numéro de dépôt: 93401552.0
(22) Date de dépôt: 17.06.1993
(51) Int. Cl.: C07K 7/18, A61K 38/08

(54) **Nouveaux dérivés peptidiques à activité antagoniste de la bradykinine, leur procédé de préparation et les compositions pharmaceutiques qui les contiennent**
Neue Peptid-Derivate mit Bradikyninantagonischer Wirkung, Verfahren zur deren Herstellung und pharmazeutische Zubereitungen die sie enthalten
New peptide derivatives with bradykinin-antagonistic activity, process to prepare them and pharmaceutical compositions containing them

(30) Priorité: 18.06.1992 FR 9207378
(43) Date de publication de la demande: 12.01.1994
(73) Titulaire: ADIR ET COMPAGNIE, F-92415 Courbevoie Cédex (FR)
(72) Inventeur: Fauchere, Jean-Luc, F-92210 Saint-Cloud (FR); Thurieau, Christophe, F-75116 Paris (FR); Paladino, Joseph, F-78700 Conflans Sainte Honorine (FR); Kucharczyk, Nathalie, F-92130 Issy les Moulineaux (FR); Canet, Emmanuel, F-75005 Paris (FR)

(56) Documents cités:
- EP-A- 0 370 453
- EP-A- 0 472 220

## Description

La présente invention concerne de nouveaux dérivés peptidiques à activité antagoniste de la bradykinine, leur procédé de préparation et les compositions pharmaceutiques qui les contiennent.

La bradykinine, nonapeptide naturel, est connue comme médiateur des réactions inflammatoires et douloureuses ainsi que dans les états d'hypotension.

La bradykinine a de plus des effets contracturants sur le muscle lisse en particulier trachéal, utérin ou intestinal. En clinique, la bradykinine a été impliquée dans la physiopathologie des états de choc des réactions inflammatoires, de l'asthme et de l'hyperréactivité bronchique, des rhinites allergiques ou virales, de la pancréatite, des arthrites, du "dumping" syndrome post-gastrectomie, du psoriasis, de l'oedème angio-neurotique héréditaire, de la migraine.

Des antagonistes de bradykinine ont été décrits dans la littérature pour le traitement de situations physiopathologiques où des concentrations élevées de bradykinine ont pu être mises en évidence comme dans les cas de choc septique (Robinson et al., Am. J. Med. 59-61, 1975), l'arthrite (Shavura et al., Arch. Int. Pharmacodyn. 262-279, 1983), d'asthme, de rhinite allergique et de nombreuses autres réactions inflammatoires et allergiques (Bradykinin antagonists : Basic and Clinical research (1991) ed R.Burch, Marcel Dekker Inc, N. York).

C'est le cas, en particulier, des composés décrits par Stewart et Vavrek dans les brevets WO 86/07263 et WO 89/01781 qui ont mis en évidence que le remplacement de la proline en position 7 de la bradykinine par une D-phénylalanine permettait d'obtenir une activité antagoniste. Les brevets EP 0370453 et EP 0413277 décrivent des antagonistes de bradykinine obtenus par remplacement de la proline en position 7 par une D-phénylalanine ou un résidu tétrahydroisoquinoléine-3-carbonyle et modification des fonctions C et N terminales du peptide.

La présente invention décrit des antagonistes de bradykinine nouveaux non seulement par les modifications originales de la position A₇ de la bradykinine, par d'autres substitutions d'amino-acides dans différentes positions mais encore par l'introduction de substituants des fonctions N et C terminales du peptide. Ces diverses modifications confèrent aux composés de l'invention des activités pharmacologiques particulièrement intenses.

Plus spécifiquement, la présente invention concerne des composés de formule générale (I) : 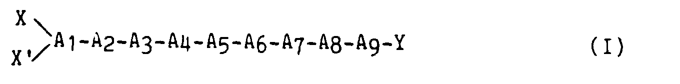 dans laquelle :
- X et X',: substituants du groupement aminé terminal du peptide de formule (I), identiques ou différents, représentent :
- un atome d'hydrogène,
- un radical 4-guanidinobenzoyle (Gba) substitué ou non sur le noyau phényle par un ou plusieurs atomes d'halogène, radicaux hydroxy, mercapto, alkyle (C₁-C₆) linéaire ou ramifié, alkylthio (C₁-C₆) linéaire ou ramifié, alkoxy (C₁-C₆) linéaire ou ramifié,
- un radical alkyle (C₁-C₆) linéaire ou ramifié,
- un radical aryle (C₆-C₁₂),
- un radical alkoxycarbonyl (C₁-C₆) linéaire ou ramifié,
- un radical alkylsulfonyl (C₁-C₆) linéaire ou ramifié,
- un radical 9-fluorénylméthyloxycarbonyl,
- un radical guanidinoacyle (C₁-C₆) linéaire ou ramifié substitué ou non par un radical amino,
- ou un radical de formule 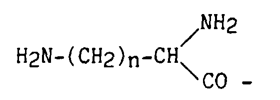 dans laquelle n est compris entre 1 et 6,
- A₁ et A₉,: identiques ou différents, représentent :
- une liaison,
- un résidu de formule 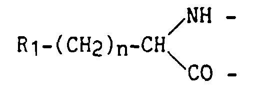 dans laquelle n représente un nombre entier compris entre 1 et 6 et R₁ un radical amino ou guanidino,
- ou n'importe quel acide aminé basique substitué ou non sur sa chaîne latérale,
- A₂ et A₃,: identiques ou différents, représentent :
- une liaison,
- un résidu proline (Pro), hydroxyproline (Hyp), 2-azabicyclo[2.2.2] octane-3-carbonyle (Abo), 2-azabicyclo[2.2.1]heptane-3-carbonyle (Abh), homoproline (hPro), 3,4-déhydroproline (dhPro), sarcosine (Sar), N-méthylphénylglycine (NMePgl), azetidine-2-carbonyle (Azt), ou alanine (Ala),
- A₄: représente une liaison ou un résidu glycine (Gly), sarcosine (Sar) ou un résidu de formule :

-NH-(CH₂)ₘ-CO-

dans laquelle m est un nombre entier compris entre 2 et 8,
- A₅: représente une liaison ou un résidu phénylalanine (Phe), β-(2-thiényl)alanine (Thi), β-cyclohexylalanine (Cha), β-naphtylalanine (Nal), β-furylalanine (Fur), β-pyranylalanine (Pra), β-pyridylalanine (Pyr),
- A₆: représente une liaison ou n'importe quel résidu d'acide aminé,
- A₇: l'un quelconque des résidus suivants : 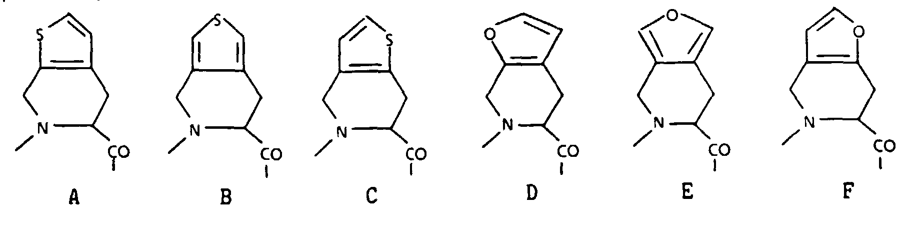 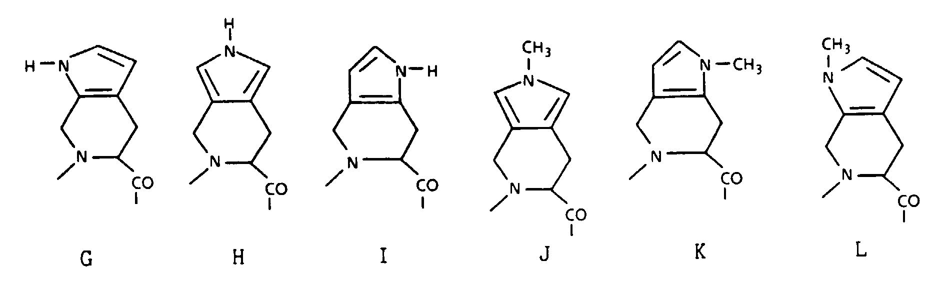 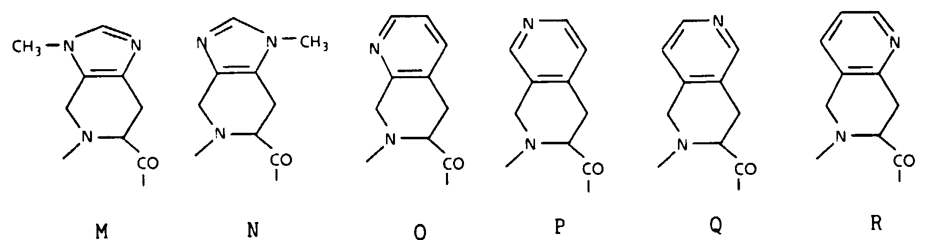 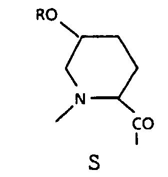 (R représente un radical (C1-C6) linéaire ou ramifié)
ou
- A₇: représente un résidu méthylphénylalanine (MePhe) ou un résidu tétrahydroisoquinoline-3-carbonyle (Tic) (à la condition que dans ce cas X représente un radical p-guanidinobenzoyle et X' un atome d'hydrogène),
- A₈: représente une liaison, un résidu octahydroindole-2-carbonyle (Oic), proline (Pro), hydroxyproline (Hyp), 2-azabicyclo[2.2.2]octane-3-carbonyle (Abo), 2-azabicyclo[2.2.1]heptane-3-carbonyle (Abh), homoproline (hPro), 3,4-déhydroproline (dhPro), sarcosine (Sar), N-méthylphénylglycine (NMePgl), azetidine-2-carbonyle (Azt), alanine ou l'un quelconque des résidus A à R définis pour A₇,
- Y,: substituant du groupement carbonyle terminal du peptide de la formule (I), représente un radical hydroxy, alkoxy (C₁-C₆) linéaire ou ramifié, amino (substitué ou non par un ou deux radicaux alkyle (C₁-C₆) linéaires ou ramifiés ou phényle -chacun des radicaux alkyle ou phényle pouvant être lui-même substitué par un radical amino, guanidino ou uréido), ou bien le groupement carboxy terminal du peptide de formule (I) est réduit en alcool correspondant (ol),
leurs sels d'addition à un acide ou à une base pharmaceutiquement acceptable,
étant entendu que :
- lorsque X représente un radical p-guanidinobenzoyle, X' un atome d'hydrogène, A₁ un résidu arginine (Arg), A₂ un résidu proline (Pro), A₃ un résidu hydroxyproline (Hyp), A₄ un résidu Glycine (Gly), A₅ un résidu β-(2-thiényl)alanine (Thi), A₆ un résidu sérine (Ser), A₈ un résidu octahydroindole-2-carbonyle (Oic), A₉ un résidu arginine (Arg) et Y un radical hydroxy alors A₇ est différent d'un résidu tétrahydroisoquinoline-3-carbonyle (Tic) de configuration D ou L au niveau de son carbone α,
- chaque liaison peptidique -CO-NH- du peptide de formule (I) peut être éventuellement remplacée par une liaison pseudopeptidique choisie parmi -CH₂-NH-, -CH₂-S-, -CH₂-SO-, -CH₂-SO₂-, -NH-CO- ou -CH=CH-,
- chaque amino-acide de la séquence peptidique étant optiquement pur et le carbone α de chaque amino-acide ayant la configuration D ou L.

Parmi les acides pharmaceutiquement acceptables on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables on peut citer l'hydroxyde de sodium, l'hydroxyde de potassium, la triéthylamine, la tertbutylamine, etc...

L'expression "n'importe quel acide aminé" utilisée dans la présente demande inclut aussi bien les acides aminés naturels que les acides aminés dits non protéiques communément utilisés par l'homme de métier dans la synthèse d'analogues synthétiques de peptides naturels.

Parmi les acides aminés naturels, on peut citer la glycine, l'alanine, la valine, la leucine, l'isoleucine, la sérine, la méthionine, la thréonine, la phénylalanine, la tyrosine, le tryptophane, la cystéine, la proline, l'histidine, l'acide aspartique, l'asparagine, la glutamine, l'acide glutamique, l'arginine, l'ornithine et la lysine.

Parmi les acides aminés non protéiques, on peut citer la norleucine, la norvaline, l'alloisoleucine, l'homoarginine, la thiaproline, la déhydroproline, l'hydroxyproline, l'homosérine, la cyclohexylglycine, la phénylglycine, l'acide amino n-butyrique, la cyclohexylalanine, l'acide aminophényl-butyrique, les phénylalanines mono, di ou tri substituées sur le groupement phényl en position ortho, meta ou para par le(s) groupement(s) choisi(s) parmi : (C₁-C₆) alkyle, (C₁-C₆) alkoxy, halogène, trifluorométhyle, nitro, amino, mono ou di (C₁-C₆) alkyl amino ou encore un groupement méthylène dioxy, β-2 et 3-thiénylalanine, β-2 et 3-furylalanine, β-2, 3 et 4-pyri-dylalanine, β-(2 et 3-benzothiényl)alanine, β-(1- et 2-naphtyl)alanine, des dérivés O-alkylés de la sérine, thréonine, tyrosine, les cystéines S-alkylées, tous les acides aminés de configuration D, isomères des acides aminés naturels de configuration L, etc.

L'invention s'étend aussi au procédé de préparation des dérivés de formule (I) qui peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B.W. ERICKSON et R.B. MERRIFIELD ("The Proteins", Solid-Phase Peptide Synthesis, 3ème édition, 257-527, 1976).

La synthèse sur phase solide peut se réaliser sur un automate qui effectue de façon répétitive et programmable des cycles de déprotection, couplage et lavages nécessaires à l'introduction séquentielle des acides aminés dans la chaîne peptidique. L'acide aminé, de préférence C-terminal est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la formation après synthèse d'une fonction C-terminale acide carboxylique, amide ou alcool.

Les acides aminés sont alors introduits un à un dans l'ordre déterminé par l'opérateur. Chaque cycle de synthèse correspondant à l'introduction d'un acide aminé, comporte une déprotection, de préférence N-terminale de la chaîne peptidique, des lavages successifs destinés à éliminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphenylphosphorylazide (DPPA).

Des activations par formation d'anhydrides mixtes sont également possibles.

Chaque acide aminé est introduit dans le réacteur en quadruple excès environ, par rapport au degré de substitution de la résine et en quantité environ équivalente par rapport aux agents de couplage. La réaction de couplage peut être vérifiée à chaque étape de la synthèse par le test de réaction à la ninhydrine décrit par E. KAISER et Coll. (Analyt. Biochem., 34, 595, 1970).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole sert à séparer le peptide de la résine ainsi qu'à libérer éventuellement le peptide de ses groupes protecteurs. Le composé est alors purifié par des techniques classiques de purification, notamment chromatographiques.

Les peptides de la présente invention peuvent être également obtenus par couplage en solution de fragments peptidiques sélectivement protégés, qui peuvent être préparés soit sur phase solide, soit en solution. L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

La synthèse de peptides contenant des liaisons pseudopeptidiques telles que -CH₂-NH-, -CH₂-S-, -CH₂-SO-, -CH₂-SO₂-, -NH-CO-, -CH=CH- est effectuée soit par les méthodes en solution soit de façon combinée avec la synthèse sur phase solide en utilisant les méthodes classiques de la chimie organique. Ainsi, par exemple, l'introduction de la liaison -CH₂-NH se fait en préparant en solution l'aldéhyde Fmoc-NH-CHR-CHO selon la technique décrite par FEHRENTZ et CASTRO (Synthesis, 676-678, 1983) et en le condensant avec la chaîne peptidique croissante soit sur phase solide selon la technique décrite par SASAKI et COY (Peptides, 8, 119-121, 1988), soit en solution.

Les composés de formule (I) possèdent des propriétés pharmacologiques très intéressantes, en particulier antagonistes de la bradykinine. A ce titre, ils peuvent être utilisés avec profit dans un certain nombre d'indications thérapeutiques comme les traumatismes, les écorchures, les brûlures, les éruptions cutanées, eczémas, érythèmes, oedèmes, angines, l'arthrite, l'asthme, les allergies, les rhinites, les chocs anaphylactiques, les inflammations, les hypotensions artérielles, les douleurs, les prurits et les insuffisances de mobilité des spermatozoïdes.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les suppositoires, crèmes, pommades, gels dermiques, les aérosols, ampoules buvables et injectables...

La posologie varie selon l'âge et le poids du patient, la nature et la sévérité de l'affection ainsi que la voie d'administration.

Celle-ci peut être orale (incluant la voie inhalée et sublinguale), nasale, rectale, parentérale ou transdermique. D'une manière générale, elle s'échelonne entre 0,1 µg/kg et 5 mg/kg pour un traitement en une ou plusieurs prises par 24 heures.

Les exemples suivants illustrent l'invention et ne la limitent en aucune façon.

Dans les exemples ci-dessous, les acides aminés dont les abréviations commencent par une lettre majuscule sont de configuration L.

Les acides aminés dont les abréviations commencent par une lettre minuscule sont de configuration D.

Les abréviations utilisées dans les exemples sont les suivantes :
- Gba: à la place de 4-guanidinobenzoyle,
- Arg: à la place du résidu arginine,
- Pro: à la place du résidu proline,
- dhPro: à la place de 3,4-déhydroproline,
- Hyp: à la place du résidu hydroxyproline,
- Gly: à la place du résidu glycine,
- Thi: à la place du résidu β-(2-thiényl)alanine,
- Ser: à la place du résidu serine,
- Tic: à la place du résidu tétrahydroisoquinoline-3-carbonyle,
- Oic: à la place du résidu octahydroindole-2-carbonyle,
- [3,2-c]ttp: à la place du résidu d'acide-(6R)-4,5,6,7-tétrahydrothiéno [3,2-c]pyridine-6-carboxylique,
- Dpr: à la place du résidu d'acide 2,3-diaminopropionique,
- Gpr: à la place du résidu d'acide 2-amino-3-guanidinopropionique,
- Dbu: à la place du résidu d'acide 2,4-diaminobutyrique,
- Sar: à la place du résidu sarcosine,
- Aoc: à la place du résidu d'acide 8-aminooctanoique,
- [3,4-c]ttp: à la place du résidu d'acide (6R)-4,5,6,7-tétrahydrothiéno [3,4-c]pyridine-6-carboxylique,
- [2,3-c]ttp: à la place du résidu d'acide (6R)-4,5,6,7-tétrahydrothiéno [2,3-c]pyridine-6-carboxylique,
- [2,3-c]tfp: à la place du résidu d'acide (6R)-4,5,6,7-tétrahydrofuro [2,3-c]pyridine-6-carboxylique,
- [3,4-c]tfp: à la place du résidu d'acide (6R)-4,5,6,7-tétrahydrofuro [3,4-c]pyridine-6-carboxylique,
- [3,2-c]tfp: à la place du résidu d'acide (6R)-4,5,6,7-tétrahydrofuro [3,2-c]pyridine-6-carboxylique,
- [2,3-c]tpp: à la place du résidu d'acide (6R)-4,5,6,7-tétrahydro-3H-pyrrolo[2,3-c]pyridine-6-carboxylique,
- [3,4-c]tpp: à la place du résidu d'acide (6R)-4,5,6,7-tétrahydro-2H-pyrrolo[3,4-c]pyridine-6-carboxylique,
- [3,2-c]tpp: à la place du résidu d'acide (6R)-4,5,6,7-tétrahydro-1H-pyrrolo[3,2-c]pyridine-6-carboxylique,
- [2m]tpp: à la place du résidu d'acide (6R)-2-méthyl-4,5,6,7-tétrahydro2H-pyrrolo[3,4-c]pyridine-6-carboxylique,
- [1m]tpp: à la place du résidu d'acide (6R)-1-méthyl-4,5,6,7-tétrahydro1H-pyrrolo[3,2-c]pyridine-6-carboxylique,
- [3m]tpp: à la place du résidu d'acide (6R)-3-méthyl-4,5,6,7-tétrahydro-3H-pyrrolo[2,3-c]pyridine-6-carboxylique,
- [3m]tip: à la place du résidu d'acide (6R)-3-méthyl-4,5,6,7-tétrahydro-3H-imidazo[5,4-c]pyridine-6-carboxylique,
- [1m]tip: à la place du résidu d'acide (6R)-1-méthyl-4,5,6,7-tétrahydro-1H-imidazo[4,5-c]pyridine-6-carboxylique,
- [1,7]tna: à la place du résidu d'acide (6R)-5,6,7,8-tétrahydro-1,7-naphtyridine-6-carboxylique,
- [2,7]tna: à la place du résidu d'acide (6R)-5,6,7,8-tétrahydro-2,7-naphtyridine-6-carboxylique,
- [3,7]tna: à la place du résidu d'acide (6R)-5,6,7,8-tétrahydro-3,7-naphtyridine-6-carboxylique,
- [4,7]tna: à la place du résidu d'acide (6R)-5,6,7,8-tétrahydro-4,7-naphtyridine-6-carboxylique,
- epc: à la place du résidu d'acide 5-éthoxypipéridine-2-carboxylique,
- Gbu: à la place du résidu d'acide 2-amino-4-guanidinobutyrique.

### Exemple 1 : Gba-Arg-dhPro-Hyp-Gly-Thi-Ser-tic-Oic-Arg-OH, hexafluoroacétate

Le composé de l'exemple 1 est synthétisé à partir de 2 g d'une résine substituée par 0,33 mmol/g de Fmoc-Arg-(Pmc)-OH et suivant le protocole répétitif suivant :

| N° opération | Fonction | Solvant/Réactif | Répétition/temps |
|---|---|---|---|
| 1 | lavage | DMF | 2 x 2 min |
| 2 | déprotection | 20 % pipéridine/DMF | 1 x 5 min |
| 3 | déprotection | 20 % pipéridine/DMF | 1 x 15 min |
| 4 | lavage | DMF | 3 x 2 min |
| 5 | lavage | dichlorométhane | 3 x 2 min |
| 6 | couplage | acide aminé protégé activé | 1 x 90 min |
| 7 | lavage | DMF | 3 x 2 min |
| 8 | lavage | isopropylique alcool | 3 x 2 min |
| 9 | lavage | dichlorométhane | 3 x 2 min |

Chacune de ces opérations effectuée dans 30 ml de solvant, sous agitation à température ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Les acides aminés protégés choisis ont été introduits dans l'ordre suivant : Fmoc-Oic-OH, Fmoc-tic-OH, Fmoc-Ser-(tBu)-OH, Fmoc-Thi-OH, Fmoc- Gly-OH, Fmoc-Hyp(tBu)-OH, Fmoc-dhPro-OH et Fmoc-Arg(Pmc)-OH.

L'activation en vue du couplage (opération 6) est obtenue à chaque cycle par dissolution de 4 équivalents (2,64 nmoles) de l'acide aminé protégé avec 360 mg d'HOBt dans 30 ml de DMF puis après 30 minutes à température ambiante, par addition de 618 mg de DCC. Cette solution est alors immédiatement introduite dans la cellule de réaction avec 10 ml de dichlorométhane.

A la fin des huits cycles correspondant à la fixation séquentielle de huit acides aminés, on a ainsi obtenu avec l'arginine C-terminale, un nonapeptide protégé sur ses chaînes latérales et fixé sur la résine. Après traitement dans des conditions identiques à celles décrites pour les cycles précédents par l'acide 4-guanidinobenzoïque, la résine est alors traitée par un mélange d'acide trifluoroacétique (18 ml), dichlorométhane (1 ml) et anisole (1 ml) pendant 90 minutes à température ambiante. Le filtrat et les solvants de lavage de la résine (3 x 20 ml dichlorométhane) sont réunis et évaporés à sec. Le produit est suspendu dans l'éther, filtré et séché puis purifié par CLHP préparative, sur colonne C18 (diamètre interne : 47 mm, longueur : 300 mm) et lyophilisé.

L'analyse du produit obtenu est réalisé après décomposition de celui-ci en acides aminés par hydrolyse dans de l'acide chlorhydrique 6N pendant 18 heures à 110°C et dosage quantitatif des acides aminés obtenus par CLHP. Cette analyse est conforme aux normes habituellement exigées.

| Analyse | Arg | Hyp | Ser | Gly | dhPro | Oic | tic |
|---|---|---|---|---|---|---|---|
| calculé | 2 | 1 | 1 | 1 | 1 | 1 | 1 |
| trouvé | 1,99 | 0,94 | 0,93 | 1,1 | 1,1 | 0,96 | 1,00 |

Spectre de masse (FAB) : MH⁺, m/z = 1307

Les exemples suivants ont été préparés en utilisant le mode opératoire décrit dans l'exemple 1.

### Exemple 2 : Gba-Arg-Pro-Hyp-Gly-Thi-Ser-[3,2-c]ttp-Oic-Arg, trifluoroacétate

La résine substituée par Fmoc-Oic-Arg(Pmc)-OH est condensée après déprotection à la pipéridine de la même façon que dans l'exemple 1 avec Fmoc-ttp-OH. Cet intermédiaire est obtenu à partir de Thi-OH en trois étapes :
- le chlorhydrate de thiénylalanine est obtenu dans une solution de méthanol saturée en HCl ;
- cyclisation du chlorhydrate de thiénylalanine dans une solution de HCHO à 40 % dans l'eau, sous agitation 2 h à 110°C puis 15 h à 60°C ;
- protection en Nα par le groupement Fluorénylméthyloxy-carbonyl (Fmoc).

| Analyse | Arg | Hyp | Ser | Gly | Pro | Oic | Thi |
|---|---|---|---|---|---|---|---|
| calculé | 2 | 1 | 1 | 1 | 1 | 1 | 1 |
| trouvé | 2,09 | 1,01 | 0,97 | 0,99 | 1,01 | 1,00 | 0,96 |

Spectre de masse (FAB) : MH⁺, m/z = 1315

### Exemple 3 : arg-Arg-Pro-Hyp-Gly-Thi-Ser-[3,2-c]ttp-Oic-Arg, trifluoroacétate

| Analyse | Arg | Hyp | Ser | Gly | Pro | Oic | Thi | [3,2-c]ttp |
|---|---|---|---|---|---|---|---|---|
| calculé | 3 | 1 | 1 | 1 | 1 | 1 | 1 | 1 |
| trouvé | 2,79 | 1,02 | 1,00 | 1,05 | 1,06 | 1,03 | 0,99 | 1,04 |

Spectre de masse (FAB) : MH⁺, m/z = 1309

### Exemple 4 : Gba-Arg-Pro-Hyp-Gly-Thi-Ser-tic-Oic-Dpr-OH

| Analyse | Arg | Hyp | Ser | Gly | Pro | Oic | tic | Thi |
|---|---|---|---|---|---|---|---|---|
| calculé | 1 | 1 | 1 | 1 | 1 | 1 | 1 | 1 |
| trouvé | 0,99 | 1,07 | 0,95 | 0,97 | 1,03 | 1,08 | 0,92 | 1,00 |

Spectre de masse (FAB) : MH⁺, m/z = 1239

### Exemple 5: Gba-Arg-Pro-Hyp-Gly-Thi-Ser-tic-Oic-Gpr-OH

| Analyse | Arg | Hyp | Tip | Oic | Pro | Gly | Ser |
|---|---|---|---|---|---|---|---|
| calculé | 1 | 1 | 1 | 1 | 1 | 1 | 1 |
| trouvé | 0,97 | 0,93 | 1,02 | 1,00 | 1,06 | 1,03 | 0,99 |

Spectre de masse (FAB) : MH⁺, m/z = 1281

### Exemple 6 : Gba-Dbu-Pro-Hyp-Cly-Thi-Ser-tic-Oic-Dbu-OH

| Analyse | Dbu | Oic | Tic | Ser | Gly | Hyp | Pro |
|---|---|---|---|---|---|---|---|
| calculé | 2 | 1 | 1 | 1 | 1 | 1 | 1 |
| trouvé | 2,03 | 1,01 | 0,99 | 0,91 | 1,07 | 1,02 | 1,03 |

Spectre de masse (FAB) : MH⁺, m/z = 1196

### Exemple 7 : Gba-Arg-Pro-Hyp-Gly-Thi-Ser-[3,4-c]ttp-Oic-Arg-OH

### Exemple 8 : Gba-Arg-Pro-Hyp-Gly-Thi-Ser-[2,3-c]ttp-Oic-Arg-OH

### Exemple 9 : Gba-Arg-Pro-Hyp-Gly-Thi-Ser-[2,3-c]tfp-Oic-Arg-OH

### Exemple 10 : Gba-Arg-Pro-Hyp-Gly-Thi-Ser-[3,4-c]tfp-Oic-Arg-OH

### Exemple 11 : Gba-Arg-Pro-Hyp-Gly-Thi-Ser-[3,2-c]tfp-Oic-Arg-OH

### Exemple 12 : Gba-Arg-Pro-Hyp-Gly-Thi-Ser-[2,3-c]tpp-Oic-Arg-OH

### Exemple 13 : Gba-Arg-Pro-Hyp-Gly-Thi-Ser-[3,4-c]tpp-Oic-Arg-OH

### Exemple 14 : Gba-Arg-Pro-Hyp-Gly-Thi-Ser-[3,2-c]tpp-Oic-Arg-OH

### Exemple 15 : Gba-Arg-Pro-Hyp-Gly-Thi-Ser-[3m]tpp-Oic-Arg-OH

### Exemple 16 : Gba-Arg-Pro-Hyp-Gly-Thi-Ser-[2m]tpp-Oic-Arg-OH

### Exemple 17 : Gba-Arg-Pro-Hyp-Gly-Thi-Ser-[1m]tpp-Oic-Arg-OH

### Exemple 18 : Gba-Arg-Pro-Hyp-Gly-Thi-Ser-[3m]tip-Oic-Arg-OH

### Exemple 19 : Gba-Arg-Pro-Hyp-Gly-Thi-Ser-[1m]tip-Oic-Arg-OH

### Exemple 20 : Gba-Arg-Pro-Hyp-Gly-Thi-Ser-[1,7]tna-0ic-Arg-OH

### Exemple 21 : Gba-Arg-Pro-Hyp-Cly-Thi-Ser-[2,7]tna-Oic-Arg-OH

### Exemple 22 : Gba-Arg-Pro-Hyp-Gly-Thi-Ser-[3,7]tna-Oic-Arg-OH

### Exemple 23 : Gba-Arg-Pro-Hyp-Gly-Thi-Ser-[4,7]tna-Oic-Arg-OH

### Exemple 24 : Gba-Arg-Pro-Hyp-Gly-Thi-Ser-epc-Oic-Arg-OH

### Exemple 25 arg-Arg-Pro-Hyp-Gly-Thi-Ser-[3,2-c]tfp-Oic-Arg-OH

### Exemple 26 : arg-Arg-Pro-Hyp-Gly-Thi-Ser-[3,4-c]tpp-Oic-Arg-OH

### Exemple 27 : arg-Arg-Pro-Hyp-Gly-Thi-Ser-[2,3-c]tpp-Oic-Arg-OH

### Exemple 28 : arg-Arg-Pro-Hyp-Gly-Thi-Ser-[3m]tpp-Oic-Arg-OH

### Exemple 29 : arg-Arg-Pro-Hyp-Gly-Thi-Ser-[2m]tpp-Oic-Arg-OH

### Exemple 30 : arg-Arg-Pro-Hyp-Gly-Thi-Ser-[1m]tip-Oic-Arg-OH

### Exemple 31 : arg-Arg-Pro-Hyp-Gly-Thi-Ser-[3,4-c]ttp-Oic-Arg-OH

### Exemple 32 : arg-Arg-Pro-Hyp-Gly-Thi-Ser-[2,3-c]ttp-Oic-Arg-OH

### Exemple 33 : Cba-Arg-Pro-Hyp-Gly-Thi-Ser-[3,2-c]ttp-Oic-Gpr-OH

### Exemple 34 : Gba-Gpr-Pro-Hyp-Cly-Thi-Ser-[3,2-c]ttp-Oic-Gpr-OH

### Exemple 35 : Gba-Dbu-Pro-Hyp-Gly-Thi-Ser-[3,2-c]ttp-Oic-Gpr-OH

### Exemple 36 : Gba-Gbu-Pro-Hyp-Gly-Thi-Ser-[3,2-c]ttp-Oic-Gpr-OH

### Exemple 37 : arg-Arg-Pro-Hyp-Gly-Phe-Ser-[3,2-c]ttp-Oic-Arg-OH

### Exemple 38 : arg-Arg-Pro-Hyp-Gly-Thi-Ser-Mephe-Oic-Arg-OH, tétrafluoroacétate

| Analyse | Arg | Pro | Hyp | Ser | Gly |
|---|---|---|---|---|---|
| calculé | 3 | 1 | 1 | 1 | 1 |
| trouvé | 3,05 | 1,05 | 1,00 | 0,98 | 0,90 |

Spectre de masse (FAB) : MH⁺, m/z = 1307

### Exemple 39 : Gba-Arg-Pro-Hyp-GlyΨ[CH₂S]Phe-Ser-tic-Oic-Arg-OH, pentafluoroacétate

| Analyse | Arg | Hyp | Ser | Pro | tic | Oic |
|---|---|---|---|---|---|---|
| calculé | 2 | 1 | 1 | 1 | 1 | 1 |
| trouvé | 2,14 | 1,02 | 0,93 | 0,93 | 1,00 | 0,98 |

Spectre de masse (FAB) : MH⁺, m/z = 1306

### Exemple 40 : Gba-Arg-Pro-Hyp-GlyΨ[CH₂S]phe-Ser-tic-Oic-Arg-OH, pentafluoroacétate

### Exemple 41 : Gba-Dpr-Pro-Hyp-Gly-Thi-Ser-tic-Oic-Dbu-OH, trifluoroacétate

| Analyse | Dbu | Oic | tic | Ser | Gly | Hyp | Pro | Dpr |
|---|---|---|---|---|---|---|---|---|
| calculé | 1 | 1 | 1 | 1 | 1 | 1 | 1 | 1 |
| trouvé | 0,98 | 1,08 | 0,98 | 0,94 | 1,02 | 0,94 | 0,98 | 1,08 |

Spectre de masse (FAB) : MH⁺, m/z = 1183

### Exemple 42 : Gba-Arg-Pro-Hyp-Sar-Thi-Ser-tic-Oic-Arg-OH, hexafluoroacétate

| Analyse | Arg | Hyp | Ser | Sar | Pro | Oic | tic |
|---|---|---|---|---|---|---|---|
| calculé | 2 | 1 | 1 | 1 | 1 | 1 | 1 |
| trouvé | 2,09 | 0,95 | 1,02 | 0,98 | 0,97 | 0,95 | 1,04 |

Spectre de masse (FAB) : MH⁺, m/z = 1323

### Exemple 43 : Gba-Arg-Pro-Hyp-Aoc-tic-Oic-Arg-OH, trifluoroacétate

| Analyse | Arg | Hyp | tic | Oic | Pro |
|---|---|---|---|---|---|
| calculé | 2 | 1 | 1 | 1 | 1 |
| trouvé | 1,81 | 1,04 | 1,03 | 1,07 | 1,05 |

Spectre de masse (FAB) : MH⁺, m/z = 1153

### ETUDE PHARMACOLOGIQUE DES DERIVES DE L'INVENTION

### Exemple 44 : Mesure in vitro de l'effet des dérivés de l'invention vis à vis de la réponse contractile du muscle lisse de la veine jugulaire de lapin induite par la bradykinine

Cette étude a été réalisée selon le protocole décrit par D. REGOLI et J. BARABE (Récepteur B₂-Pharmacology of Bradykinin and related kinins, Pharm. Rev., 32, 1, 1-46, 1980). Des anneaux de veine jugulaire sont suspendus dans des chambres d'organes (40 ml) contenant une solution de KREBS thermostatée à 37°C, oxygénée en permanence par un bullage de 95 % 02/5 % CO₂. La force isométrique développée par les anneaux est mesurée et enregistrée. La tension de base est d'environ 2 g. Les cuves reçoivent les concentrations du dérivé de l'invention à tester (10⁻⁸M à 10⁻⁵M), une cuve contrôle reçoit le solvant. Une courbe concentration réponse à la bradykinine est réalisée de 10⁻¹⁰M à 10⁻⁵M avec une progression par demi-log. La puissance des antagonistes à la bradykinine est mesurée à partir des courbes log dose-effet permettant la détermination des pA₂ (logarithme négatif de la concentration molaire du dérivé de l'invention qui oblige à doubler la concentration de bradykinine pour obtenir le même effet). Lors de ce test, les dérivés de l'invention ont un pA₂ compris entre 9 et 10. Plus particulièrement, le pA₂ du composé de l'exemple 2 est égal à 9,1 ± 0,1, celui de l'exemple 3 est égal à 9,4 ± 0,1, celui de l'exemple 5 est égal à 9,3 ± 0,1.

### Exemple 45 : Mesure in vivo de l'activité anti-inflammatoire des dérivés de l'invention

L'activité anti-inflammatoire des dérivés de l'invention a été mesurée en utilisant le modèle de l'oedème de la patte de rat induite par injection de carragénine (WINTER G. A. et coll. Proc. Soc. Exp. Biol. Med., 3, 544-547, 1960).
Cette étude est effectuée sur rats mâles de 180-210 g par lot de 8. Les dérivés de l'invention sont administrés par voie I.V. au temps 0 précédant immédiatement l'injection de carragénine par voie sous-cutanée dans la plante de la patte arrière droite du rat (Carragénine lambda type IV, sigma, solution à 1 %, volume injecté 0,1 ml). Le volume de la patte aux différents temps de l'expérimentation est mesurée par plethysmographie. L'inhibition de l'oedème au temps 3 heures est calculée par rapport à un lot contrôle ayant reçu le véhicule et exprimée en pourcentage d'inhibition.
Dans ces conditions, les composés des exemples 2 et 3 engendrent une inhibition de 36 % au temps 1 heure à la dose de 0,1 mg/kg I.V., le composé de l'exemple 5 engendre une inhibition de 55 % au temps 1 heure à la même dose.

### Exemple 46 : Composition pharmaceutique Pommade à 5 µg/ml du peptide de l'exemple 1

| | |
|---|---|
| Gba-Arg-dhPro-Hyp-Gly-Thi-Ser-tic-Oic-Arg-OH | 50 mg |
| Polyéthylène glycol Q.S.P. | 100 ml |

### Exemple 47 :

Soluté injectable

| | |
|---|---|
| Gba-Arg-dhPro-Hyp-Gly-Thi-Ser-tic-Oic-Arg-OH | 0,5 mg |
| Eau distillée pour préparations injectables Q.S.P. | 25 ml |

## Revendications

1. Composés de formule (I) : 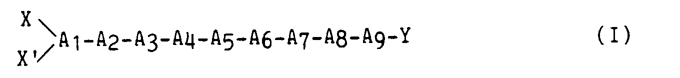 dans laquelle :
X et X', substituants du groupement aminé terminal du peptide de formule (I), identiques ou différents, tels que :
X représente :
- un radical 4-guanidinobenzoyle (Gba) substitué ou non sur le noyau phényle par un ou plusieurs atomes d'halogène, radicaux hydroxy, mercapto, alkyle (C₁-C₆) linéaire ou ramifié, alkylthio (C₁-C₆) linéaire ou ramifié, alkoxy (C₁-C₆) linéaire ou ramifié,
ou, un radical guanidinoacyle (C₁-C₆) linéaire ou ramifié substitué ou non par un radical amino,
X' représente un atome d'hydrogène,
A₁ et A₉, identiques ou différents, représentent :
- un résidu de formule 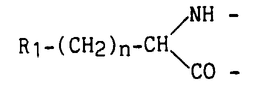 dans laquelle n représente un nombre entier compris entre 1 et 6 et R₁ un radical amino ou guanidino,
A₂ représente :
- un résidu proline (Pro), ou 3,4-déhydroproline (dhPro),
A₃ représente un résidu hydroxyproline (Hyp),
A₄ représente un résidu glycine (Gly),
A₅ représente β-(2-thiényl)alanine (Thi),
A₆ représente un résidu sérine (Ser),
A₇ l'un quelconque des résidus suivants : 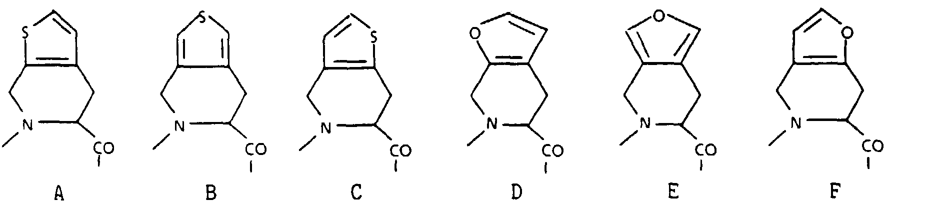 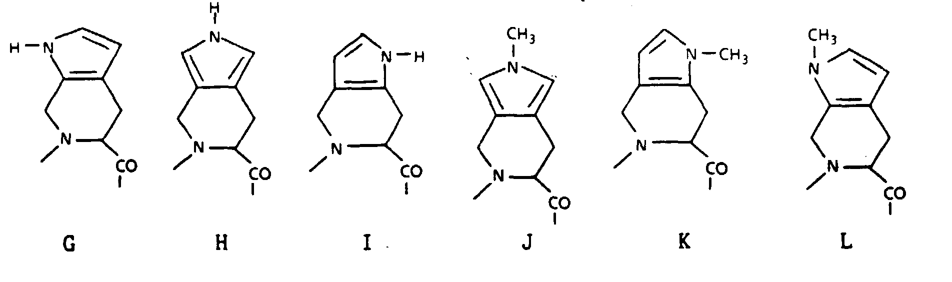 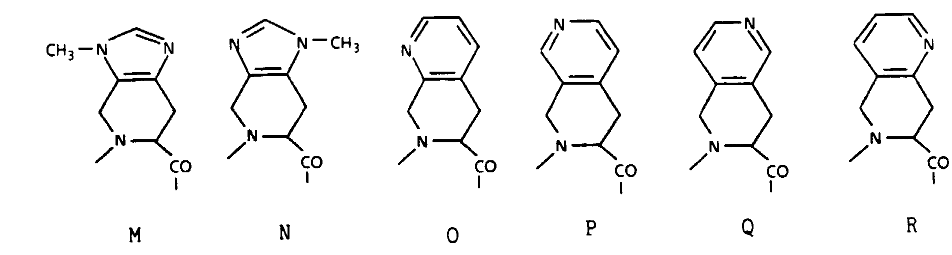 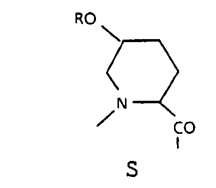 (R représente un radical (C₁-C₆) linéaire ou ramifié)
ou
A₇ représente un résidu méthylphénylalanine (MePhe) ou un résidu tétrahydroisoquinoline-3-carbonyle (Tic) à la condition que dans ce cas X représente un radical p-guanidinobenzoyle et X' un atome d'hydrogène,
A₈ représente, un résidu octahydroindole-2-carbonyle (Oic),
Y, substituant du groupement carbonyle terminal du peptide de la formule (I), représente un radical hydroxy,
leurs sels d'addition à un acide ou à une base pharmaceutiquement acceptable,
étant entendu que :
- lorsque X représente un radical p-guanidinobenzoyle, X' un atome d'hydrogène, A₁ un résidu arginine (Arg), A₂ un résidu proline (Pro), A₃ un résidu hydroxyproline (Hyp), A₄ un résidu Glycine (Gly), A₅ un résidu β-(2-thiényl)alanine (Thi), A₆ un résidu sérine (Ser), A₈ un résidu octahydroindole-2-carbonyle (Oic), A₉ un résidu arginine (Arg) et Y un radical hydroxy alors A₇ est différent d'un résidu tétrahydroisoquinoline-3-carbonyle (Tic) de configuration D ou L au niveau de son carbone α,
- chaque liaison peptidique -CO-NH- du peptide de formule (I) peut être éventuellement remplacée par une liaison pseudopeptidique choisie parmi -CH₂-NH-, -CH₂-S-, -CH₂-SO-, -CH₂-SO₂-, -NH-CO- ou -CH=CH-,
- chaque amino-acide de la séquence peptidique étant optiquement pur et le carbone α de chaque amino-acide ayant la configuration D ou L.

2. Composés de formule (I) selon la revendication 1 tels que X représente un radical 4-guanidinobenzoyle (Gba) et X' un atome d'hydrogène.

3. Composés de formule (I) selon la revendication 1 tels que A₇ représente l'un quelconque des résidus suivants : 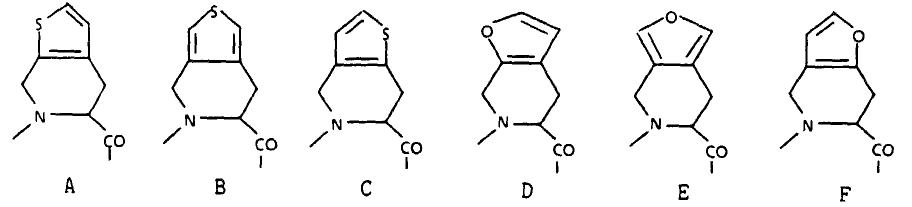 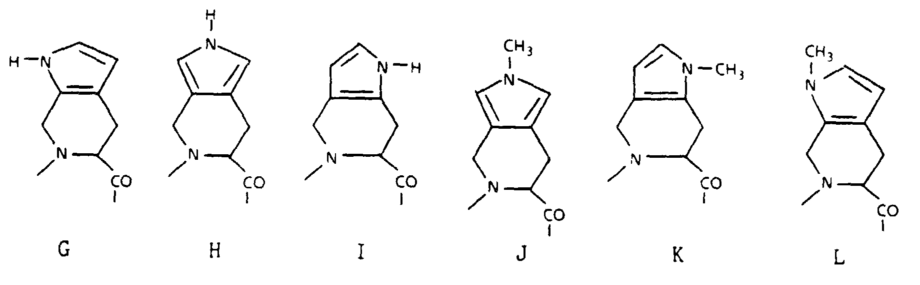 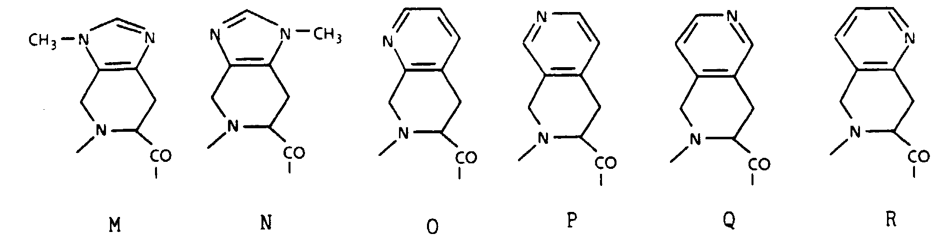 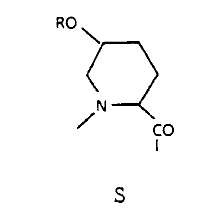 (R représente un radical (C₁-C₆) linéaire ou ramifié)

4. Composés de formule (I) selon la revendication 3 tels que X représente un radical 4-guanidinobenzoyle (Gba) et X' un atome d'hydrogène.

5. Composés de formule (I) selon la revendication 3 tels que X représente un radical arginyle et X' un atome d'hydrogène.

6. Procédé de préparation des composés de formule (I) selon la revendication 1 caractérisé en ce qu'ils peuvent être obtenus par synthèse séquentielle sur phase solide à partir d'amino-acides protégés, par synthèse à partir de fragments peptidiques en solution,
éventuellement par combinaison de ces deux techniques,
et si on le souhaite par introduction,
selon les techniques classiques de la chimie organique, d'une liaison pseudopeptidique à tout moment de synthèse de la séquence peptidique,
dérivés de formule (I) que l'on transforme, si nécessaire, en leurs sels d'addition à un acide ou à une base pharmaceutiquement acceptable.

7. Compositions pharmaceutiques contenant comme principe actif au moins un composé selon l'une quelconque des revendications 1 à 5, seul ou en combinaison avec un ou plusieurs excipients ou véhicules pharmaceutiquement acceptables.

8. Compositions pharmaceutiques selon la revendication 7 contenant au moins un principe actif selon l'une quelconque des revendications 1 à 6 utiles en tant qu'antagonistes de bradykinine dans le traitement des traumatismes d'origine variée, des troubles inflammatoires, de la douleur, des états de choc, des troubles allergiques et de l'insuffisance de mobilité des spermatozoïdes.

## Patentansprüche

1. Verbindungen der Formel (I): 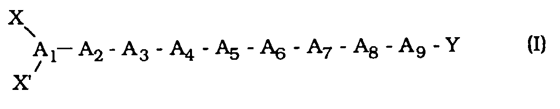 in der:
X und X', die gleichartig oder verschieden sind, Substituenten der endständigen Aminogruppe des Peptids der Formel (I), wie:
X:
- eine 4-Guanidinobenzoylgruppe (Gba), die gegebenenfalls am Phenylkern durch eines oder mehrere Halogenatome, Hydroxylgruppen. Mercaptogruppen, geradkettige oder verzweigte (C₁-C₆)-Alkylgruppen, geradkettige oder verzweigte (C₁-C₆)-Alkylthiogruppen oder geradkettige oder verzweigte (C₁-C₆)-Alkoxygruppen substituiert ist, oder
- eine geradkettige oder verzweigte, gegebenenfalls durch eine Aminogruppe substituierte (C₁-C₆)-Guanidinoacylgruppe,
X' ein Wasserstoffatom.
A₁ und A₉, die gleichartig oder verschieden sein können:
- einen Rest der Formel 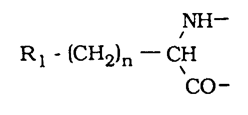 in der n eine ganze zahl mit einem Wert zwischen 1 und 6 und R₁ eine Aminogruppe oder eine Guanidinogruppe darstellen,
A₂ einen Prolinrest (Pro) oder einen 3,4-Dehydroprolinrest (dhPro),
A₃ einen Hydroxyprolinrest (Hyp),
A₄ einen Glycinrest (Gly),
A₅ einen β-(2-Thienyl)-alaninrest (Thi),
A₆ einen Serinrest (Ser),
A₇ einen der folgenden Reste: 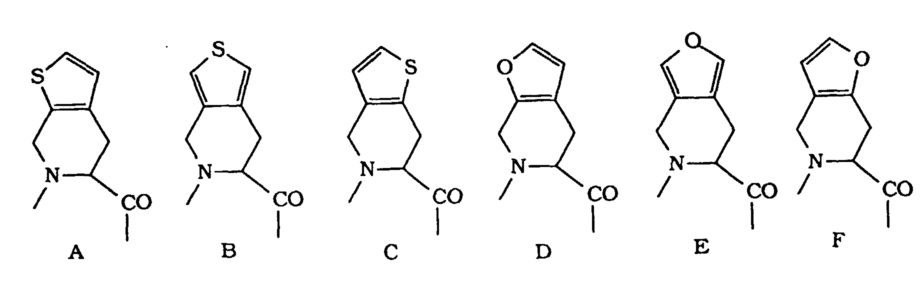 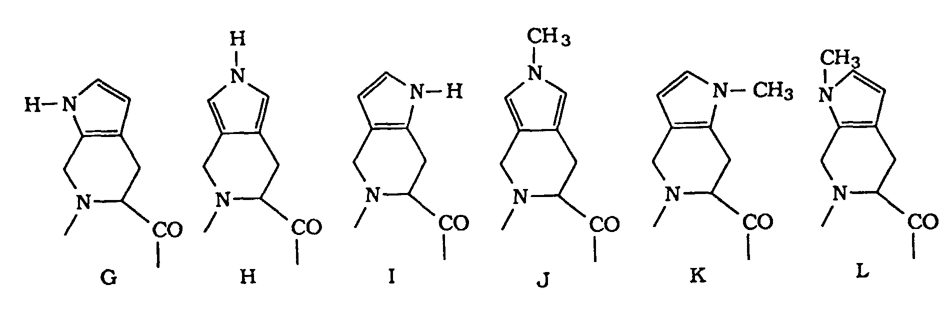 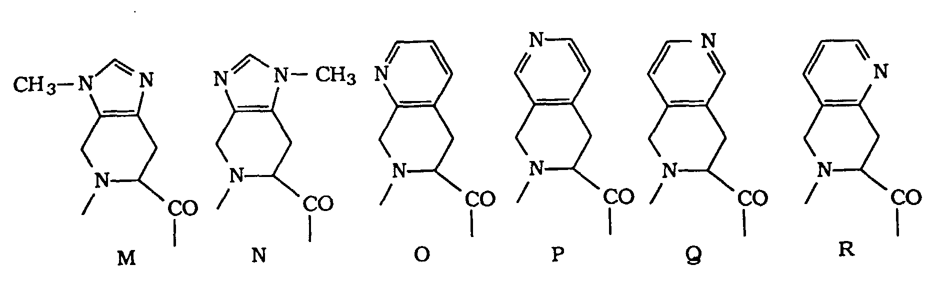 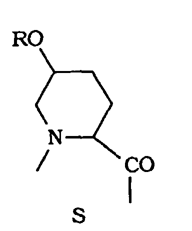 (worin R eine geradkettige oder verzweigte (C₁-C₆)-Alkyl-Gruppe darstellt), oder
A₇ einen Methylphenylalaninrest (MePhe) oder einen Tetrahydroisochinolin-3-carbonylrest (Tic) mit der Maßgabe, daß in diesem Fall X eine p-Guanidinobenzoylgruppe und X' ein Wasserstoffatom darstellen,
A₈ einen Octahydroindol-2-carbonylrest (Oic) und
Y, der Substituent der endständigen Carbonylgruppe des Peptids der Formel (I), eine Hydroxylgruppe bedeuten.
und
deren Additionssalze mit einer pharmazeutisch annehmbaren Säure oder Base. mit der Maßgabe, daß:
- wenn X eine p-Guanidinobenzoylgruppe, X' ein Wasserstoffatom. A einen Argininrest (Arg), A₂ einen Prolinrest (Pro), A₃ einen Hydroxyprolinrest (Hyp), A₄ einen Glycinrest (Gly), A₅ einen β-(2-Thienyl)-alaninrest (Thi). A₆ einen Serinrest (Ser), A₈ einen Octahydroindol-2-carbonylrest (Oic), A₉ einen Argininrest und Y einen Hydroxylrest bedeuten, A₇ von dem Tetrahydroisochinolin-3-carbonylrest (Tic) in der Konfiguration D oder L im Bereich seines α-Kohlenstoffatoms verschieden ist,
- jede Peptidbindung -CO-NH- des Peptids der Formel (I) gegebenenfalls durch eine Pseudopeptidbindung, ausgewählt aus -CH₂-NH-, -CH₂-S-, -CH₂-SO-, -CH₂-SO₂- -NH-CO- oder -CH=CH- ersetzt sein kann, und
- jede Aminosäure der Peptidsequenz optisch rein ist und das α-Kohlenstoffatom einer jeden Aminosäure in der Konfiguration D oder L vorliegt.

2. Verbindungen der Formel (I) nach Anspruch 1, worin X eine 4-Guanidinobenzoylgruppe (Gba) und X' ein Wasserstoffatom bedeuten.

3. Verbindungen der Formel (I) nach Anspruch 1, worin A₇ einen der folgenden Rest darstellt: 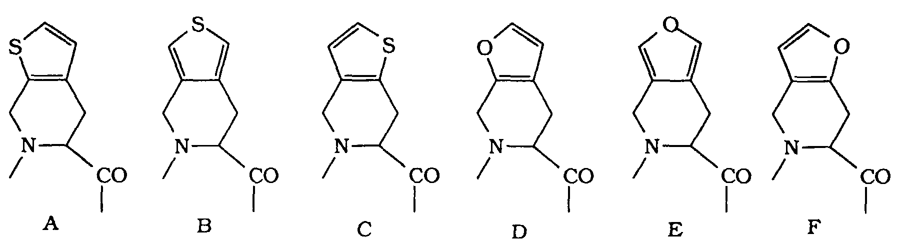 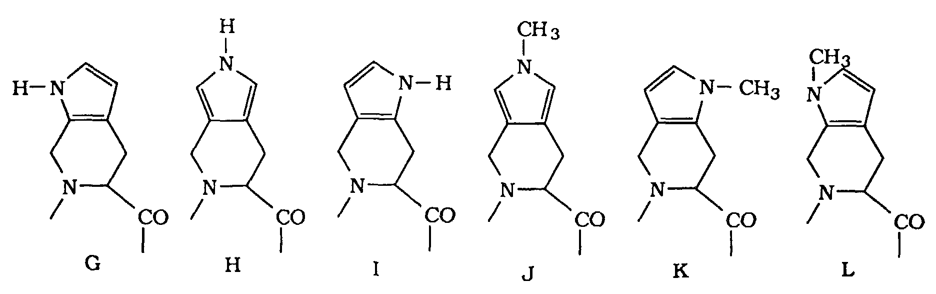 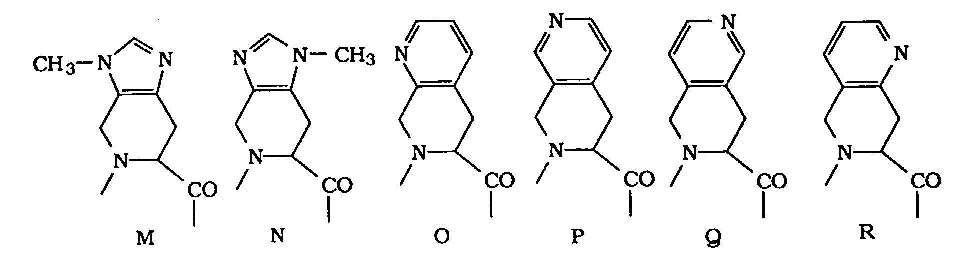 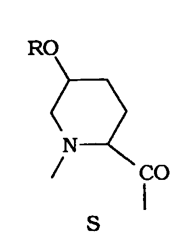 (worin R eine geradkettige oder verzweigte (C₁-C₆)-Alkyl-Gruppe bedeutet).

4. Verbindungen der Formel (I) nach Anspruch 3, worin X eine 4-Guanidinobenzoylgruppe (Gba) und X' ein Wasserstoffatom bedeuten.

5. Verbindungen der Formel (I) nach Anspruch 3, worin X einen Arginylrest und X' ein Wasserstoffatom bedeuten.

6. Verfahren zur Herstellung der Verbindungen der Formel (I) nach Anspruch 1, **dadurch gekennzeichnet**, daß sie durch sequentielle Synthese auf fester Phase ausgehend von geschützten Aminosäuren, durch Synthese in Lösung ausgehend von Peptidfragmenten oder gegebenenfalls durch einen Kombination dieser beiden Techniken hergestellt werden können und man in jedem Augenblick der Synthese der Peptidsequenz gewünschtenfalls mit Hilfe klassischer Methoden der organischen Chemie eine Pseudopeptidbindung einführen kann, welche Derivate der Formel (I) man erforderlichenfalls in ihre Additionssalze mit einer pharmazeutisch annehmbaren Säure oder Base umwandelt.

7. Pharmazeutische Zubereitungen enthaltend als Wirkstoff mindestens eine Verbindung nach einem der Ansprüche 1 bis 5, allein oder in Kombination mit einem oder mehreren, pharmazeutisch annehmbaren Trägermaterialien oder Bindemitteln.

8. Pharmazeutische Zubereitungen nach Anspruch 7, enthaltend mindestens einen Wirkstoff nach einem der Ansprüche 1 bis 5 zur Verwendung als Bradykinin-Antagonisten bei der Behandlung von traumatischen Zuständen unterschiedlichen Ursprungs, Entzündungsstörungen, von Schmerz, von Schockzuständen, von allergischen Störungen und von der Insuffizienz der Beweglichkeit von Spermatozoiden.

## Claims

1. Compounds of formula (I): 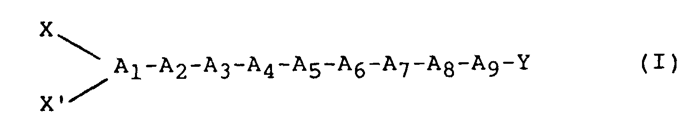 wherein:
X and X', substituents of the terminal amine group of the peptide of formula (I) and being the same or different, such that:
- X represents:
- a 4-guanidinobenzoyl (Gba) radical that is unsubstituted or substituted on the phenyl ring by one or more halogen atoms, hydroxy, mercapto, linear or branched (C₁-C₆)-alkyl, linear or branched (C₁-C₆)-alkylthio, linear or branched (C₁-C₆)-alkoxy radicals, or
- a linear or branched (C₁-C₆)-guanidinoacyl radical that is unsubstituted or substituted by an amino radical,
X' represents a hydrogen atom,
A₁ and A₉, which may be the same or different, represent:
- a residue of the formula 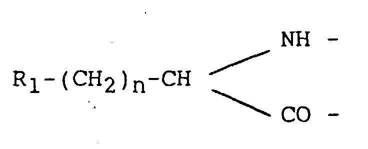 wherein n represents an integer from 1 to 6 and R₁ represents an amino or guanidino radical,
A₂ represents a proline (Pro) or a 3,4-dehydroproline (dhPro) residue,
A₃ represents a hydroxyproline (Hyp) residue,
A₄ represents a glycine (Gly) residue,
A₅ represents β-(2-thienyl)alanine (Thi),
A₆ represents a serine (Ser) residue,
A₇ represents any one of the following residues: 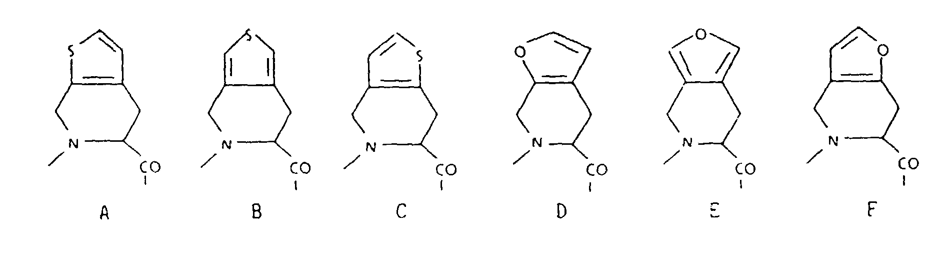 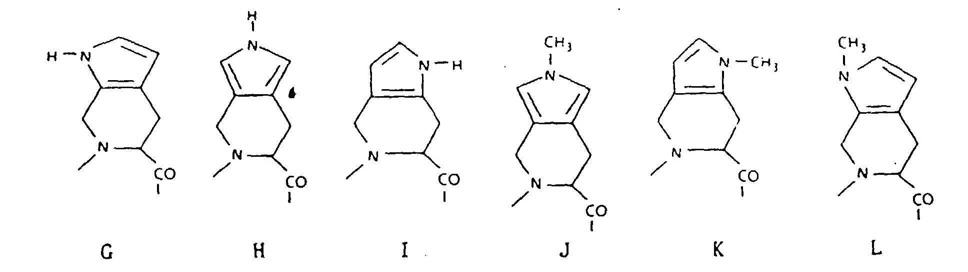 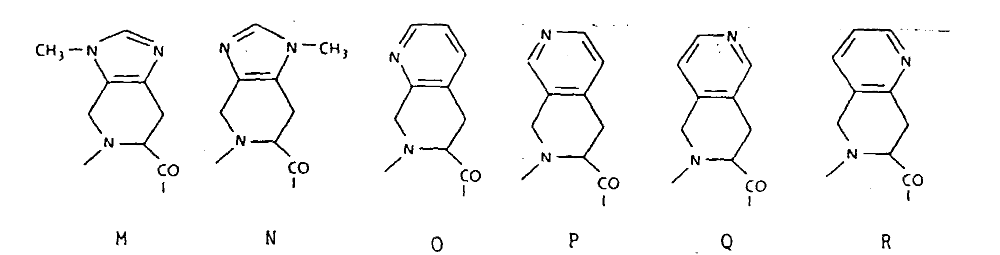 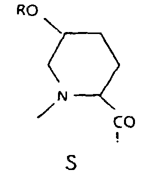 (R represents a linear or branched (C₁-C₆)alkyl radical)
or
A₇ represents a methylphenylalanine (MePhe) residue or a tetrahydroisoquinoline-3-carbonyl (Tic) residue with the proviso that in that case X represents a p-guanidinobenzoyl radical and X' represents a hydrogen atom,
A₈ represents an octahydroindole-2-carbonyl (Oic) residue,
Y, a substituent of the terminal carbonyl group of the peptide of formula (I), represents a hydroxy radical,
addition salts thereof with a pharmaceutically acceptable acid or base,
on the understanding that:
- when X represents a p-guanidinobenzoyl radical, X' represents a hydrogen atom, A₁ represents an arginine (Arg) residue, A₂ represents a proline (Pro) residue, A₃ represents a hydroxyproline (Hyp) residue, A₄ represents a glycine (Gly) residue, A₅ represents a β-(2-thienyl)alanine (Thi) residue, A₆ represents a serine (Ser) residue, A₈ represents an octahydroindole-2-carbonyl (Oic) residue, A₉ represents an arginine (Arg) residue and Y represents a hydroxy radical, then A₇ is other than a tetrahydroisoquinoline-3-carbonyl (Tic) residue having the D or L configuration in respect of its α-carbon,
- each peptide bond -CO-NH- of the peptide of formula (I) can optionally be replaced by a pseudo-peptide bond selected from -CH₂-NH-, -CH₂-S-, -CH₂-SO-, -CH₂-SO₂-, -NH-CO- and -CH=CH-,
- each amino acid of the peptide sequence being optically pure and the α-carbon of each amino acid having the D or L configuration.

2. Compounds of formula (I) according to claim 1 wherein X represents a 4-guanidinobenzoyl (Gba) radical and X' represents a hydrogen atom.

3. Compounds of formula (I) according to claim 1 wherein A₇ represents any one of the following residues: 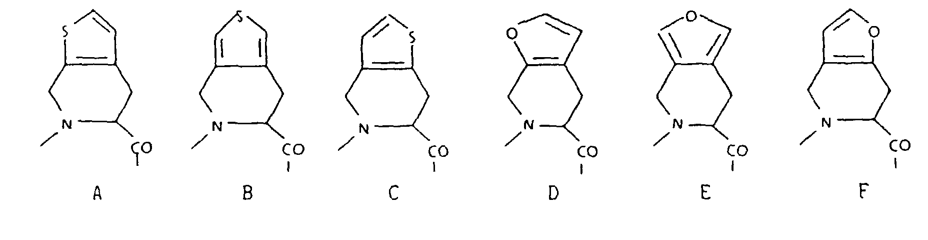 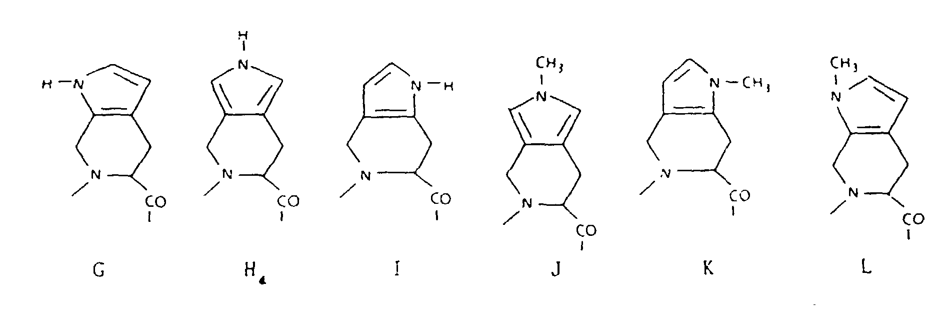 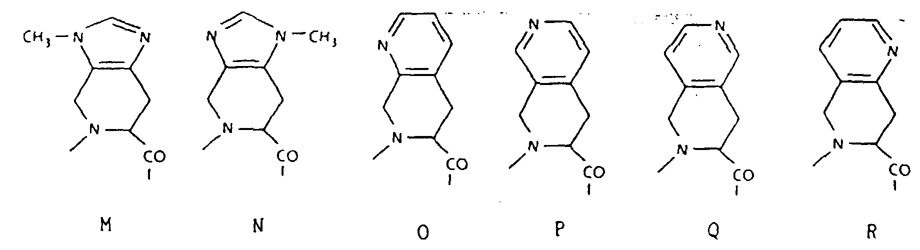 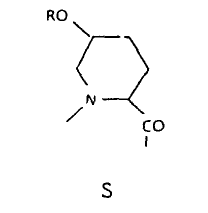 (R represents a linear or branched (C₁-C₆)alkyl radical).

4. Compounds of formula (I) according to claim 3 wherein X represents a 4-guanidinobenzoyl (Gba) radical and X' represents a hydrogen atom.

5. Compounds of formula (I) according to claim 3 wherein X represents an arginyl radical and X' represents a hydrogen atom.

6. Process for the preparation of the compounds of formula (I) according to claim 1, characterised in that they can be obtained by solid-phase sequential synthesis starting from protected amino acids, by synthesis starting from peptide fragments in solution, optionally by a combination of those two techniques, and, if desired, by the introduction, in accordance with conventional techniques of organic chemistry, of a pseudo-peptide bond at any time in the synthesis of the peptide sequence,
which compounds of formula (I) are, if necessary, converted into their addition salts with a pharmaceutically acceptable acid or base.

7. Pharmaceutical compositions comprising as active ingredient at least one compound according to any one of claims 1 to 5, on its own or in combination with one or more pharmaceutically acceptable excipients or carriers.

8. Pharmaceutical compositions according to claim 7 comprising at least one active ingredient according to any one of claims 1 to 5 for use as antagonists of bradykinin in the treatment of trauma of varying origin, inflammatory disorders, pain, states of shock, allergic disorders and insufficient mobility of spermatozoa.
